# EUROPEAN PATENT SPECIFICATION

(11) **EP 0 705 272 B1**
(45) Date of publication and mention of the grant of the patent: **27.03.2002**
(21) Application number: 93913459.9
(22) Date of filing: 24.06.1993
(51) Int. Cl.: C07J 73/00, A61K 31/58

(54) **NOVEL PROCESS FOR PREPARING 17 BETA-SUBSTITUTED 4-AZAANDROSTANE DERIVATIVES**
NEUES VERFAHREN ZUM HERSTELLEN VON 17 BETA-SUBSTITUIERTEN 4-AZAANDROSTAN-DERIVATEN
NOUVEAU PROCEDE DE PREPARATION DE DERIVES DE 4-AZAANDROSTANE A SUBSTITUTION EN POSITION 17 BETA

(43) Date of publication of application: 10.04.1996
(73) Proprietor: RICHTER GEDEON VEGYESZETI GYAR R.T., 1475 Budapest (HU)
(72) Inventor: TUBA, Zoltán, H-1022 Budapest (HU); HORVATH, Judit, H-1084 Budapest (HU); SZELES, János, H-1096 Budapest (HU); KOLLAR, Lászlú, H-8200 Veszprém (HU); BALOGH, Gábor, H-1181 Budapest (HU)
(74) Representative: Pett, Christopher Phineas
(86) International application number: HU9300039
(87) International publication number: WO9500531

(56) References cited:
- EP-A- 0 004 949
- EP-A- 0 473 225

## Description

This invention relates to a novel process for preparing 17β-substituted 4-azaandrostane derivatives of general formula (I) 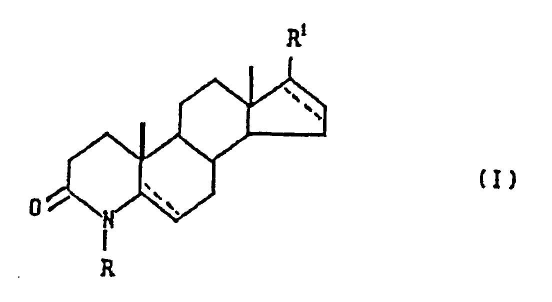 wherein
- R: represents hydrogen or a C₁₋₃alkyl group;
- R¹: represents a carboxamido group mono- or disubstituted by straight or branched chain C₁₋₈alkyl group(s); or a free carboxyl group; or a carboxyl group esterified with a straight or branched chain C₁₋₅ alcohol; and the
- ----: bond line represents a single or double bond;
as well as their salts formed with pharmaceutically acceptable bases when R¹ is a free carboxyl group.

The compounds of general formula (I) inhibit the 5α-reductase enzyme and therefore, they block the transformation of testosterone to dihydrotestosterone. Thus, the compounds of general formula (I) are useful for the healing of dihydrotestosterone-dependent diseases, e.g. prostatic hyperplasia, acne vulgaris, seborrhoea or female hirsutism.

According to literature references [European patent No. 4,949; as well as J. Med. Chem. 27, pages 1690 to 1701 (1984)] the preparation of the known compounds of general formula (I) can be accomplished in the manner described hereinafter.

After reacting pregnenolone (3β-hydroxy-5-pregnen-20-one) with elemental iodine in pyridine, then the "21-pyridinium iodide" obtained is cleaved at the bond between C₂₀ and C₂₁ by using sodium methoxide to obtain the corresponding "17-carbomethoxy derivative". The obtained 3β-hydroxy-17β-carbomethoxyandrost-5-ene is oxidized by aluminum isopropoxide in the presence of cyclohexanone in toluene, subsequently the carbomethoxy group is hydrolyzed to the carboxylic acid and transformed to the "17-carboxylic acid chloride" by using oxalyl chloride. This acyl chloride is converted to e.g. "17β-(N,N-diethylcarbamoyl)" derivative by using diethylamine. After oxidizing the thus obtained 17β-(N,N-diethylcarbamoyl)androst-4-en-3-one to "seco acid" by using sodium periodate in tertiary butanol in the presence of potassium permanganate, the seco compound is reacted with ammonia or an other primary amine in ethylene glycol to obtain e.g. 3-oxo-4-methyl-4-aza-androst-5-ene-17β-(N,N-diethylcarboxamide). This latter substance is hydrogenated to the corresponding "4-aza-5α-androstane" derivative in glacial acetic acid in the presence of hydrogen and platinum oxide catalyst. After isolation the final products are purified in various ways.

The starting substance of the process described in the literature is pregnenolone, which is obtained by hydrogenating the double bond in position 16 of pregnadienolone acetate obtained by the decomposition of diosgenin or solasodin of natural origin. However, the availability of pregnenolone is decreasing because the Dioscorea species growing wild in Mexico is on the verge of dying off. The root of this plant is used for extracting diosgenin. On the other hand, the cultivation of Solanum aviculare and the isolation of solasodin therefrom are not economical according to our experience.

Considering their therapeutic activity, there exists a continuous demand on the target compounds in the pharmaceutical industry, however, this demand is more and more difficult to satisfy by using the known process of preparation because those discussed above.

The aim of the present invention is to develop a preparation process from a starting material which is easily available. From this point of view the new 17--halogeno-4-azaandrostene derivatives of general formula (II) 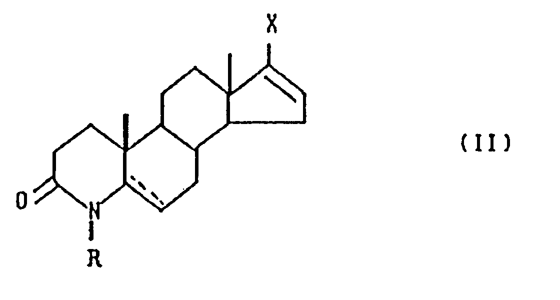 proved to be suitable.

In Advanced Organic Chemistry, J. March, 4th Edition, pp 484-486, the methods for alkoxycarbonyl-de-hydrogenation are reviewed. Using different CO-binding catalysts and reaction mixtures, R-carboxylic acids or their esters or amides may be prepared (here R represents simple alkyl or aryl groups). A large number of suitable catalysts are discussed.

Surprisingly, it has been found that a process completely satisfying the above demands for preparing the target compounds of general formula (I) can be accomplished by
reacting a 17-halogeno-4-azaandrostene derivative of general formula (II), wherein R and the ---- bond line are as defined above, and X is iodine, with a primary or secondary alkylamine containing a C₁₋₈alkyl group or a straight or branched chain C₁₋₅ alcohol, respectively in dimethylformamide or dimethylsulfoxide medium in the presence of a palladium(II) salt and phosphines or a palladium(II) complex and a tertiary amine base in carbon monoxide atmosphere at a temperature between 35 °C and 80 °C,
then, if desired, hydrogenating a compound of general formula (I) containing a double bond as ---- bond line in the presence of a catalyst to obtain a compound of general formula (I) containing a single bond as ---- bond line, and/or
hydrolyzing in a known way a thus obtained compound of general formula (I) containing an esterified carboxyl group as R¹ to obtain a compound of general formula (I) containing a free carboxyl group as R¹, and/or
transforming a thus obtained compound of general formula (I) containing a free carboxyl group as R¹ to its salt by reacting it with a pharmaceutically acceptable base.

According to a preferred embodiment of the invention a compound of general formula (II) is reacted with a primary or secondary amine in dimethylformamide in the presence of palladium(II) acetate, triphenylphosphine and triethylamine under carbon monoxide at 60 °C for 90 to 120 minutes. After the reaction has become complete, the amines and dimethylformamide are distilled off under reduced pressure, the residue is dissolved in chloroform and successively washed with water, aqueous hydrochloric acid solution, aqueous sodium hydrogen carbonate solution and again with water until neutral. After drying and evaporating the solvent, the residue is purified by chromatography or recrystallization or by using both methods.

If desired, the double bond [being in position 16 or in positions 5 and 16 depending on the starting compound of general formula (II)] of the obtained compounds of general formula (I) containing a mono- or disubstituted carboxamido group as R¹ may be hydrogenated in the presence of gaseous hydrogen and charcoal supported palladium catalyst in formic acid or in the presence of hydrogen and platinum oxide catalyst in glacial acetic acid.

For preparing compounds of general formula (I) containing an alkoxycarbonyl group as R¹, a compound of general formula (II) is preferably reacted with a C₁₋₅ alkanol in dimethylsulfoxide, in the presence of a mixture of palladium(II) acetate, 1,4-bis(diphenylphosphino)butane and triethylamine under carbon monoxide at 60 °C for 10 to 15 hours. After the reaction has become complete, the volatile components are distilled off under reduced pressure, the residue is dissolved in chloroform and the water-soluble components are removed by washing with water. After drying the solution and evaporating the solvent, the residue is purified by chromatography or recrystallization or by using both methods.

If desired, the obtained compound of general formula (I) is hydrogenated as described above, i.e. in formic acid in the presence of hydrogen and charcoal supported palladium or in glacial acetic acid in the presence of hydrogen and platinum oxide as catalyst and/or optionally hydrolyzed to the corresponding 17β--carboxylic acid derivative in alkaline medium.

In the process according to the invention 1,4-bis(diphenylphosphino)butane, 1,2-bis(diphenylphosphino)ethane, triphenylphosphine or 1,3-bis(diphenylphosphino)propane is preferably used as a phosphine although a complex of the above phosphines with palladium(II) salts may also be employed: e.g. the reaction is carried out at 35 to 60 °C with primary or secondary amines and at 40 to 80 °C with C₁₋₅ alcohols in the presence of bis(triphenylphosphino)palladium(II) dichloride.

The process according to the invention provides the use of the easily available 3-keto- Δ⁴ derivatives as starting substances, which can be obtained by the decomposition of sitosterin. According to the invention the building-up of the 17-carboxamido or 17-carboalkoxy group, respectively can safely be carried out and the scale increase of the process needed for the industrial utilization is not burdened by any problem.

The starting substances used in the process according to the invention such as "4-aza-17-hydrazone" derivatives as well as the compounds of general formula II are new. Similarly, the 5,16-diunsaturated 4-aza-17-carboxamido- as well as 4-aza-17-alkoxycarbonyl derivatives of general formula (I) are also novel compounds. The saturated 4-aza-17-carboxamido derivatives as well as the saturated 4-aza-17-methoxycarbonyl derivative are known from the literature [European patent No. 4,949; J. Med. Chem. 27, pages 1690 to 1701 (1984)].

The novel 17-halogeno-4-azaandrostene derivatives of general formula (II) used as starting substances in the process of the present invention can be prepared as follows.

4-Aza-5α-androstane-3,17-dione, 4-methyl-4-aza-androstane-3,17-dione as well as 4-azaandrost-5-ene-3,17-dione (hereinafter named as 4-aza-17-keto derivatives), which are known compounds, can be prepared by a process described in the literature [J. Pharm. Sci. 63, pages 19 to 23 (1974); J. Med. Chem. 27, pages 1690 to 1701 (1984); J. Org. Chem. 46, pages 1442 to 1446 (1981)] from the known 17β-hydroxy-androst-4-en-3-one.

The known "4-aza-17-keto derivatives" are reacted with hydrazine hydrate in ethanol in the presence of triethylamine, after working up the reaction mixture (carried out as described in Example 1) the "hydrazone derivatives" formed are isolated and the crude products are immediately used without any purification for preparing the 17-halogeno-4-azaandrostene derivatives of general formula (II) as described hereinafter.

For the preparation of 17-iodo-4-azaandrostene derivatives the "hydrazone derivatives" are dissolved in chloroform or benzene or in a mixture thereof or in tetrahydrofuran and then reacted with elemental iodine in the presence of a tertiary amine base at room temperature. After complete reaction the compounds of general formula (II) are obtained as described in Example 4.

The process according to the invention is illustrated in detail by the following non-limiting Examples.

### Example 1

### Preparation of 17-hydrazono-4-aza-5α-androstan-3-one

To a suspension containing 10 g (0.0346 mol) of 4-aza-5α-androstane-3,17-dione in 100 ml of ethanol 14 ml (0.1 mol) of triethylamine and 50.ml (1.0 mol) of hydrazine hydrate are added and the mixture is boiled under reflux for 3 hours. (The progress of the reaction is followed by thin layer chromatography.) After the reaction has become complete the reaction mixture is cooled down, the solution is evaporated to one tenth of its original volume and the product is precipitated by adding about a 10-fold volume of water. After compaction the precipitate is filtered, washed with water until neutral and dried to obtain the title compound. Yield: 9.44 g (90%), m.p.: 254-258 °C.
¹H-NMR (300 MHz, CDCl₃) δ ppm: 0.86 (s,3H,18-CH₃), 0.93 (s,3H,19-CH₃), 2.41 (m,2H,H-2), 3.07 (dd,1H,H-5), 4.77 (br,2H,NH₂), 5.74 (br,1H,NH).

### Example 2

### Preparation of 17-hydrazono-4-azaandrost-5-en-3-one

The process described in Example 1 is followed, except that 4-azaandrost-5-ene-3,17-dione is used as starting substance to obtain the title compound.
Yield: 35%, m.p.: 379-382 °C.
IR (KBr) ν: 1633 (C=C), 1661 (C=N), 1693 (C=O), 3200 (NH), 3350 (NH₂) cm⁻¹.

### Example 3

### Preparation of 17-hydrazono-4-methyl-4-aza-5α-androstan-3-one

The process described in Example 1 is followed, except that 4-methyl-4-aza-5α-androstane-3,17-dione is used as starting substance to give the title compound.
Yield: 75%, m.p.: 211-218 °C.
¹H-NMR (300 MHz, CDCl₃) δ ppm: 0.86 (s,3H,18-CH₃), 0.91 (s,3H,19-CH₃), 2.93 (s,3H,N-CH₃), 3.05 [dd(J=3.6; J=12.6), 1H, H-5], 4.78 (v br, 2H,NH₂).

### Example 4

### Preparation of 17-iodo-4-aza-5α-androst-16-en-3-one

After dissolving 9.1 g (0.03 mol) of 17-hydrazono-4--aza-5α-androstan-3-one in 1200 ml of an 1:1 chloroform/benzene mixture and adding 90 ml of triethylamine, 11.4 g (0.045 mol) of iodine dissolved in 110 ml of benzene are dropwise added to the above solution. The reaction mixture is stirred at room temperature for additional 60-90 minutes. (The progress of the reaction is followed by thin layer chromatography). After complete occurrence of the reaction the obtained solution is diluted with 500 ml of chloroform and successively washed with 10% aqueous hydrochloric acid solution, water, 5% aqueous sodium thiosulfate solution, water, 5% aqueous sodium hydrogen carbonate solution, finally with water and dried over anhydrous sodium sulfate. After evaporating the solvents under reduced pressure the residue is purified by chromatography on a silica gel column by using first chloroform and subsequently a 95:5 chloroform/acetone mixture as eluents. The product obtained is recrystallized from ethanol to give the title compound. Yield: 5.9 g (50%), m.p.: 278-282 °C.
¹H-NMR (300 MHz, CDCl₃) δ ppm: 0.73 (s,3H,18-CH₃), 0.91 (s,3H,19-CH₃), 3.1 (dd,1H,H-5), 6.18 (m,1H,H-16), 6.9 (br, 1H, NH).

### Example 5

### Preparation of 17-iodo-4-azaandrosta-5,16-dien-3-one

The process described in Example 4 is followed, except that 17-hydrazono-4-azaandrost-5-en-3-one is used as starting substance to obtain the title compound. Yield: 57%, m.p.: 227-230 °C.
¹H-NMR (300 MHz, CDCl₃) δ ppm: 0.78 (s,3H,18-CH₃), 1.13 (s,3H,19-CH₃), 4.9 [dd(J=2.4; J=5.1),1H,H-6], 6.15 [dd(J=3.2; J=1.7),1H,H-16), 8.27 (br,1H,NH).

### Example 6

### Preparation of 17-iodo-4-methyl-4-aza-5α-androst-16-en-3-one

The process described in Example 4 is followed, except that 17-hydrazono-4-methyl-4-aza-5α-androstan-3-one is used as starting substance and the reaction is carried out in benzene. The title compound is obtained in a yield of 52%, m.p.: 176-181 °C.
¹H-NMR (300 MHz, CDCl₃) δ ppm: 0.74 (s, 3H/18-CH₃) , 0.92 (s,3H,19-CH₃), 2.94 (s,3H,N-CH₃), 3.07 [dd(J=3.7; J=12.6), 1H, H-5], 6.13 [dd(J=3.2; J=1.7),1H,H-16].

### Example 7

### Preparation of 3-oxo-4-aza-5α-androst-16-ene-17β-(N-tert-butylcarboxamide)

To a solution containing 3.99 g (0.01 mol) of 17-iodo-4-aza-5α-androst-16-en-3-one in 150 ml of dimethylformamide, 0.224 g (0.001 mol) of palladium(II) acetate, 0.524 g (0.002 mol) of triphenylphosphine, 10 ml of triethylamine and 15 ml (0.14 mol) of tert-butylamine are added and the mixture is heated at 60 °C under carbon monoxide for 90 to 120 minutes. (The progress of the reaction is followed by thin layer and gas chromatography.) After the reaction has become complete the amines and dimethylformamide are distilled off under reduced pressure, then the residue is dissolved in 150 ml of chloroform and successively washed with water, 5% aqueous hydrochloric acid solution, saturated aqueous sodium hydrogen carbonate solution and saturated aqueous sodium chloride solution until neutral and finally dried over anhydrous sodium sulfate. After evaporating the solvent the residue is purified by chromatography on a silica gel column by using ethyl acetate as eluent to obtain the title compound. Yield: 3.16 g (85%), m.p.: 292-297 °C.
¹H-NMR (300 MHz, CDCl₃) δ ppm: 0.93 (s,3H,19-CH₃), 1.0 (s,3H,18-CH₃), 1.4 (s,3H,C(CH₃)₃), 2.15 (m,2H,H-15a+H-15b), 2.4 (m,2H,H-2), 3.08 [dd (J=4.5; J=7.0),1H,H-5], 5.48 (br s,1H,NH), 5.6 (br s,1H,NH), 6.18 [dd (J=1.7; J=1.4),1H,H-16].

### Example 8

### Preparation of 3-oxo-4-aza-5α-androst-16-ene-17β-[N-(2,2-dimethylpropyl)carboxamide]

The process described in Example 7 is followed by using 17-iodo-4-aza-5α-androst-16-en-3-one as starting substance and 2,2-dimethylpropylamine (neopentylamine) as reactant to obtain the title compound. Yield: 82%.
¹H-NMR (300 MHz, CDCl₃) δ ppm: 0.92 (s,9H,C(CH₃)₃), 0.95 (s,3H,19-CH₃), 1.02 (s,3H,18-CH₃), 2.4 (m,2H,H-2), 3.1 (m,3H, NCH₂, H-5), 5.66 (br s,1H,NH), 5.85 (br s,1H,NH), 6.3 (br s,1H, H-16).

### Example 9

### Preparation of 4-methyl-3-oxo-4-aza-5α-androst-16-ene-17-carboxylic acid methyl ester

A mixture containing 0.41 g (0.001 mol) of 17-iodo-4-methyl-4-aza-5α-androst-16-en-3-one, 0.0224 g (0.1 mmol) of palladium(II) acetate, 0.0213 g (0.05 mmol) of 1,4-bis(diphenylphosphino)butane, 0.3 ml of triethylamine, 2 ml of methanol and 15 ml of dimethylsulfoxide is stirred under carbon monoxide at 60 °C for 10 to 15 hours. (The progress of the reaction is followed by thin layer and gas chromatography.) After complete reaction the mixture is evaporated under reduced pressure, the residue is dissolved in 15 ml of chloroform, the chloroform solution is washed 4 times with water and dried over anhydrous sodium sulfate. After evaporation of the solvent the residue is purified by chromatography on a silica gel column by using an 1:10 mixture of ethyl acetate/petroleum ether as eluent. The title compound is obtained in a yield of 0.014 g (40%), m.p.: 182-186 °C.
¹H-NMR (300 MHz, CDCl₃) δ ppm: 0.93 (s,6H,18-CH₃ + + 19-CH₃), 2.45 (m,2H,H-2), 2.94 (s,3H,NCH₃), 3.07 (dd,1H,H-5), 3.72 (s,3H,OCH₃), 6.76 (br s,1H,H-16).

### Example 10

### Preparation of 3-oxo-4-aza-5α-androst-16-ene-17-carboxylic acid methyl ester

The process described in Example 9 is followed by using 17-iodo-4-aza-5α-androst-16-en-3-one as starting substance to give the title compound. Yield: 42%,
m.p.: 270 °C.
¹H-NMR (300 MHz, CDCl₃) δ ppm: 0.92 (s,3H,19-CH3), 0.94 (s,3H,18-CH₃), 2.4 (m,2H,H-2), 3.07 (dd,1H,H-5), 3.72 (s,3H,OCH₃), 6.15 (br s,1H,NH), 6.75 (br s,1H,H-16).

### Example 11

### Preparation of 3-oxo-4-azaandrosta-5,16-diene-17β-(N-tert-butylcarboxamide)

The process described in Example 7 is followed by using 17-iodo-4-azaandrosta-5,16-dien-3-one as starting substance and tert-butylamine as reactant to give the title compound. Yield: 78%, m.p.: 266-269 °C.
¹H-NMR (300 MHz, CDCl₃) δ ppm: 1.04 (s,3H,18-CH₃), 1.14 (s,3H,19-CH₃), 1.38 (s,9H,C(CH₃)₃), 2.5 (m,2H,H-2), 4.88 [dd (J=2.1; J=2.7), 1H,H-6], 5.5 (br s,1H,NH), 6.2 [dd (J=1.8; J=0.9),1H,H-16], 8.08 (br s,1H,NH).

### Example 12

### Preparation of 4-methyl-3-oxo-4-aza-5α-androst-16-ene-17β-(N,N-diethylcarboxamide)

The process described in Example 7 is followed by using 17-iodo-4-methyl-4-aza-5α-androst-16-en-3-one as starting substance and diethylamine as reactant. In this way the title compound is obtained in a yield of 84%, m.p.: 205-210 °C.
¹H-NMR (300 MHz, CDCl₃) δ ppm: 0.93 (s, 3H, 19-CH3) , 1.09 (s,3H,18-CH₃), 1.13 (t,6H,N(CH₂CH₃)₂), 2.94 (s,3H,NCH₃), 3.06 (dd,1H,H-5), 5.26 (m,1H,H-16).

### Example 13

### Preparation of 4-methyl-3-oxo-4-aza-5α-androstane-17β- (N,N-diethylcarboxamide)

A suspension containing 1 g of charcoal supported palladium catalyst in 6 ml of water is added to the solution of 1 g (2.6 mmol) of 4-methyl-3-oxo-4-aza-5α-androst-16-ene-17β-(N,N-diethylcarboxamide) in 40 ml of formic acid under nitrogen. The heterogeneous mixture is stirred at room temperature for 4 to 5 hours while observing the progress of the reduction by thin layer chromatography. After the reaction has become complete the catalyst is filtered off and washed with an 1:1 mixture of chloroform/methanol. After evaporating the combined solution to dryness the evaporation residue is thoroughly triturated with water, the precipitate is filtered and washed with water to obtain the title compound. Yield: 0.88 g (87%), m.p.: 180-181 °C (after recrystallyzation from ethyl acetate).

### Example 14

### Preparation of 3-oxo-4-aza-5α-androstane-17β-(N-tert-butylcarboxamide)

The process described in Example 13 is followed by using 3-oxo-4-aza-5α-androst-16-ene-17β-(N-tert-butylcarboxamide) as starting substance to obtain the title compound. Yield: 90%, m.p.: 283-286 °C.

### Example 15

### Preparation of 3-oxo-4-aza-5α-androstane-17β-carboxylic acid methyl ester

The process described in Example 13 is followed by using 3-oxo-4-aza-5-androst-16-ene-17-carboxylic acid methyl ester as starting substance to give the title compound. Yield: 85%, m.p.: 301-304 °C (after recrystallization from ethyl acetate).

### Example 16

### Preparation of 3-oxo-4-aza-5α-androstane-17β-(N-tert-butylcarboxamide)

The process described in Example 13 is followed by using 3-oxo-4-azaandrosta-5,16-ene-17β-(N-tert-butylcarboxamide) as starting substance to obtain the title compound. Yield: 70%, m.p.: 283-286 °C.

## Claims

1. A process for the preparation of 17β-substituted 4-azaandrostane derivatives of the general formula (I), 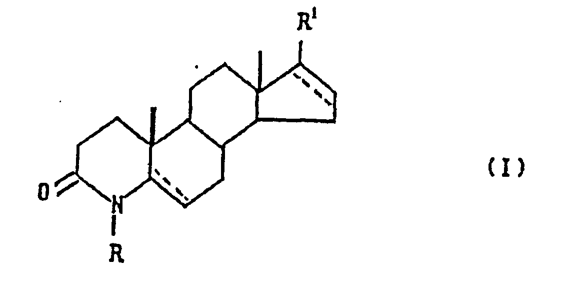 wherein
R represents hydrogen or a C₁₋₃alkyl group;
R¹ represents a carboxamido group mono- or disubstituted by straight or branched chain C₁₋₈alkyl group(s); or a free carboxyl group; or a carboxyl group esterified with a straight or branched chain C₁₋₅ alcohol; and the
---- bond line represents a single or double bond;
as well as their salts formed with pharmaceutically acceptable bases when R¹ is a free carboxyl group, **which comprises,**
reacting a 17-halogeno-4-azaandrostene derivative of general formula (II), 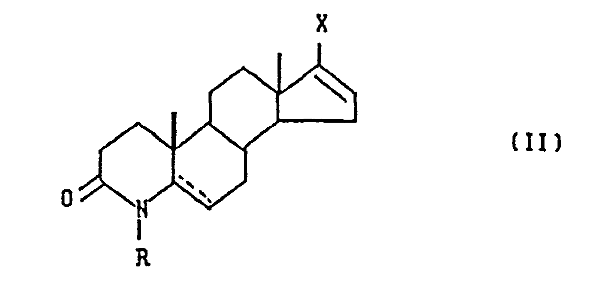 wherein R and the ---- bond line are as defined above, and X is iodine, with a primary or secondary alkylamine containing a C₁₋₈alkyl group or a straight or branched chain C₁₋₅ alcohol, respectively in dimethylformamide or dimethylsulfoxide medium in the presence of a palladium(II) salt and phosphines or a palladium(II) complex and a tertiary amine base in carbon monoxide atmosphere at a temperature between 35 °C and 80 °C,
then, if desired, hydrogenating an obtained compound of general formula (I) containing a double bond as ---- bond line, wherein R and R¹ are as defined for the general formula (I), in the presence of a catalyst to obtain a compound of general formula (I) containing a single bond as ---- bond line, wherein R and R¹ are as defined for the general formula (I), and/or
hydrolyzing in a known way a thus obtained compound of general formula (I) containing an esterified carboxyl group as R¹, wherein R and the ---- bond line are as defined for the general formula (I), to obtain a compound of general formula (I) containing a free carboxyl group as R¹, wherein R and the ---- bond line are as defined for the general formula (I), and/or
transforming a thus obtained compound of general formula (I), wherein R and the ---- bond line are as defined for the general formula (I) and R¹ is a free carboxyl group, to its salt by reacting it with a pharmaceutically acceptable base.

2. A process as claimed in claim 1, **which comprises** using triethylamine as a tertiary amine base.

3. A process as claimed in claim 1 or claim 2, **which comprises,** using palladium(II) acetate or palladium(II) chloride as palladium(II) salt.

4. A process as claimed in any of the claims 1 to 3, **which comprises** using triphenylphosphine, 1,4-bis(diphenylphosphino)butane, 1,2-bis(diphenylphosphino)ethane or 1,3-bis(diphenylphosphino)propane as phosphine.

5. A process as claimed in claim 1 or claim 2, **which comprises** using bis(triphenylphosphino)palladium(II) dichloride or diacetate as palladium(II) complex.

6. A process as claimed in any of the claims 1 to 5, **which comprises** carrying out the amidation or alkoxycarbonylation reaction at a temperature of 50 to 60 °C.

7. A process as claimed in any of the claims 1 to 6, **which comprises** carrying out the saturation of the double bonds in the presence of a palladium or platinum catalyst.

8. A process as claimed in claim 7, **which comprises** carrying out the hydrogenation in glacial acetic acid or formic acid medium.

9. A process as claimed in any of the claims 1 to 8 for the preparation of compounds of the general formula (I) containing an N,N-diethylcarboxamido, N-tert-butylcarboxamido or N-(2,2-dimethylpropyl)carboxamido group as R¹, **which comprises** using diethylamine, tertiary butylamine or 2,2-dimethylpropylamine, respectively as an alkylamine.

10. A process as claimed in any of the claims 1 to 8 for the preparation of compounds of the general formula (I) containing a methoxycarbonyl group as R¹, **which comprises** using methanol as an alcohol.

## Patentansprüche

1. Verfahren für die Herstellung von 17β-substituierten 4-Azaandrostan-Derivaten mit der allgemeinen Formel (I), 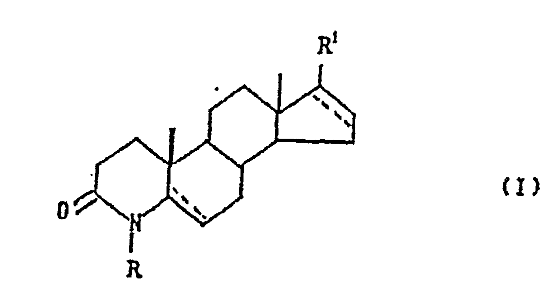 wobei
R Wasserstoff oder eine C₁₋₃-Alkylgruppe darstellt,
R¹ eine Carboxamido-Gruppe darstellt, die durch unverzweigtkettige oder verzweigtkettige C₁₋₅-Alkylgruppen mono- oder disubstituiert ist, oder eine freie Carboxylgruppe oder eine Carboxylgruppe, die mit einem unverzweigtkettigen oder verzweigtkettigen C₁₋₅-Alkohol verestert ist, und die Bindungslinie
---- eine Einfach- oder Doppelbindung darstellt,
sowie deren Salze, die mit pharmazeutisch zulässigen bzw. brauchbaren Basen gebildet sind, wenn R¹ eine freie Carboxylgruppe ist, mit folgenden Schritten:
Umsetzen eines 17-Halogen-4-azaandrosten-Derivats mit der allgemeinen Formel (II) 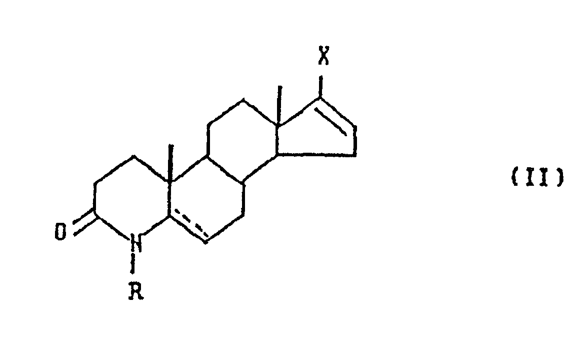 wobei R und die Bindungslinie ---- wie vorstehend definiert sind und X Iod ist, mit einem primären oder sekundären Alkylamin, das eine C₁₋₈-Alkylgruppe aufweist, bzw. mit einem unverzweigtkettigen oder verzweigtkettigen C₁₋₅-Alkohol in Dimethylformamid- oder Dimethylsulfoxid-Medium in der Gegenwart eines Palladium(II)-Salzes und eines Phosphins oder eines Palladium(II)-Komplexes und einer tertiären Aminbase in einer Kohlenmonoxid-Atmosphäre bei einer Temperatur zwischen 35 °C und 80 °C,
anschließend, falls gewünscht, Hydrieren einer erhaltenen Verbindung mit der allgemeinen Formel (I), die eine Doppelbindung als Bindungslinie ---- enthält, wobei R und R¹ wie für die allgemeine Formel (I) definiert sind, in der Gegenwart eines Katalysators, um eine Verbindung mit der allgemeinen Formel (I) zu erhalten, die eine Einfachbindung als Bindungslinie ---- enthält, wobei R und R¹ wie für die allgemeine Formel (I) definiert sind, und/oder
Hydrolysieren einer somit erhaltenen Verbindung mit der allgemeinen Formel (I), die eine veresterte Carboxylgruppe als R¹ enthält, wobei R und die Bindungslinie ---- wie für die allgemeine Formel (I) definiert sind, um eine Verbindung mit der allgemeinen Formel (I) zu erhalten, die eine freie Carboxylgruppe als R¹ enthält, wobei R und die Bindungslinie ---- wie für die allgemeine Formel (I) definiert sind, auf eine bekannte Weise und/oder
Umsetzen einer somit erhaltenen Verbindung mit der allgemeinen Formel (I), wobei R und die Bindungslinie ---- wie für die allgemeine Formel (I) definiert sind und R¹ eine freie Carboxylgruppe ist, zu ihrem Salz durch Reaktion mit einer pharmazeutisch zulässigen bzw. brauchbaren Base.

2. Verfahren nach Anspruch 1, das die Verwendung von Triethylamin als tertiäre Aminbase umfaßt.

3. Verfahren nach Anspruch 1 oder Anspruch 2, das die Verwendung von Palladium(II)acetat oder Palladium(II)chlorid als Palladium(II)-Salz umfaßt.

4. Verfahren nach einem der Ansprüche 1 bis 3, das die Verwendung von Triphenylphosphin, 1,4-Bis(diphenylphosphino)butan, 1,2-Bis(diphenylphosphino)ethan oder 1,3-Bis(diphenylphosphino)propan als Phosphin umfaßt.

5. Verfahren nach Anspruch 1 oder Anspruch 2, das die Verwendung von Bis(triphenylphosphino)palladium(II)dichlorid oder -diacetat als Palladium(II)-Komplex umfaßt.

6. Verfahren nach einem der Ansprüche 1 bis 5, das das Durchführen der Amidierungs- oder Alkoxycarbonylierungsreaktion bei einer Temperatur von 50 bis 60 °C umfaßt.

7. Verfahren nach einem der Ansprüche 1 bis 6, das das Durchführen der Sättigung der Doppelbindung in der Gegenwart eines Palladium- oder Platin-Katalysators umfaßt.

8. Verfahren nach Anspruch 7, das das Durchführen der Hydrierung in Eisessig- oder Ameisensäure-Medium umfaßt.

9. Verfahren nach einem der Ansprüche 1 bis 8 für die Herstellung von Verbindungen mit der allgemeinen Formel (I), die eine N,N-Diethylcarboxamido-, N-tert-Butylcarboxamido- oder N-(2,2-Dimethylpropyl)carboxamido-Gruppe als R¹ enthält, das eine Verwendung von Diethylamin, tertiärem Butylamin oder 2,2-Dimethylpropylamin als Alkylamin umfaßt.

10. Verfahren nach einem der Ansprüche 1 bis 8 für die Herstellung von Verbindungen mit der allgemeinen Formel (I), die eine Methoxycarbonylgruppe als R¹ enthält, das eine Verwendung von Methanol als Alkohol umfaßt.

## Revendications

1. Procédé pour la préparation de dérivés 4-azaandrostane 17β-substitué de formule générale I 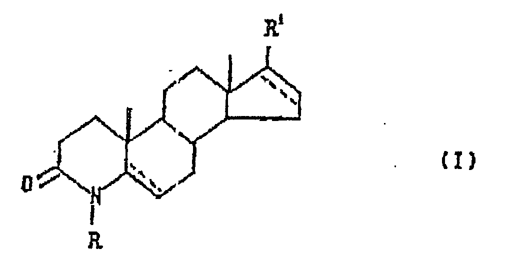 dans laquelle
- R représente un atome d'hydrogène ou un groupe alkyle en C₁-C₃ ;
- R₁ représente un groupe carboxamido mono-ou disubstitué par un ou plusieurs groupes alkyle en C₁-C₈ linéaire ou ramifié ; ou un groupe carboxylique libre ; ou un groupe carboxyle estérifié par une chaîne linaire ou ramifiée en C₁-C₅ de type alcool ; et la liaison --- représente une liaison double ou simple ;
ainsi que leurs sels formés avec des bases pharmaceutiquement acceptables lorsque R¹ représente un groupe carboxylique libre,
ledit procédé comprenant les étapes consistant à :
- faire réagir un dérivé 17-halo-4-azaandrostène de formule générale II 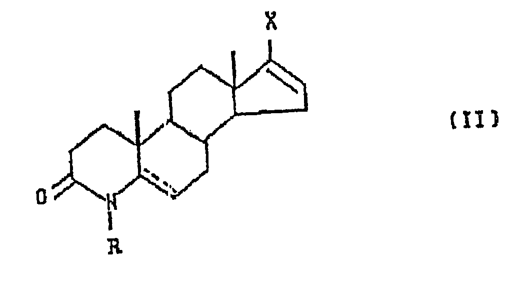 dans laquelle R et la liaison --- sont tels que définis ci-dessus, et X est un atome d'iode, avec une alkylamine primaire ou secondaire contenant un groupe alkyle en C₁-C₈ ou une chaîne linéaire ou ramifiée en C₁-C₅ de type alcool, respectivement dans un milieu diméthylformamide ou diméthylsulfoxyde en présence d'un sel de palladium (II) et de phosphines ou d'un complexe du palladium (II) et d'une base de type amine tertiaire, sous une atmosphère de monoxyde de carbone à une température comprise entre 35°C et 80°C,
- puis, si nécessaire, hydrogéner un composé de formule générale (I) ainsi obtenu comprenant une double liaison en tant que liaison ---, dans lequel R et R¹ sont tels que définis pour la formule générale (I), en présence d'un catalyseur de façon à obtenir un composé de formule générale (I) contenant une liaison simple en tant que liaison ---, dans lequel R et R¹ sont tels que définis pour la formule générale (I), et/ou
- hydrolyser, de façon connue en soi, un composé de formule générale (I) ainsi obtenu contenant un groupe carboxylique estérifié en tant que R¹,dans lequel R et la liaison --- sont tels que définis pour la formule générale (I), de façon à obtenir un composé de formule générale (I) contenant un groupe carboxylique libre en tant que R^{1,} dans lequel R et la liaison --- sont tels que définis pour la formule générale (I), et/ou
- transformer un composé de formule générale (I) ainsi obtenu, dans lequel R et la liaison --- sont tels que définis pour la formule générale (I) et R¹ est un groupe carboxylique libre, en son sel par réaction avec une base pharmaceutiquement acceptable.

2. Procédé selon la revendication 1 dans lequel la triéthylamine est utilisée comme base du type amine tertiaire.

3. Procédé selon la revendication 1 ou 2 dans lequel on utilise, en tant que sel du palladium (II), l'acétate de palladium (II),ou le chlorure de palladium (II).

4. Procédé selon l'une quelconque des revendications 1 à 3, dans lequel on utilise, comme phosphine, la triphénylphosphine, le 1,4-bis(diphénylphosphino)butane, le 1,2-bis(diphénylphosphino)éthane ou le 1,3-bis(diphénylphosphino)propane.

5. Procédé selon la revendication 1 ou 2 dans lequel on utilise, comme complexe du palladium (II), le dichlorure ou le diacétate de bis(triphénylphosphino)palladium (II).

6. Procédé selon l'une quelconque des revendications 1 à 5 dans lequel on réalise l'amidification ou l'alcoxycarbonylation à une température de 50 à 60°C.

7. Procédé selon l'une quelconque des revendications 1 à 6 dans lequel on réalise la saturation des doubles liaisons en présence d'un catalyseur au platine.

8. Procédé selon la revendication 7 dans lequel on réalise l'hydrogénation en milieu acide acétique glacial ou acide formique.

9. Procédé selon l'une quelconque des revendications 1 à 8 pour la préparation de composés de formule générale (I) contenant un groupe N,N-diéthylcarboxamido, N-tert-butylcarboxamido ou N-(2,2-diméthylpropyl)carboxamido en tant que groupe R¹, dans lequel on utilise respectivement en tant qu'alkylamine, de la diéthylamine, de la butylamine tertiaire ou de la 2,2-diméthylpropylamine.

10. Procédé selon l'une quelconque des revendications 1 à 8 pour la préparation de composés de formule générale (I) contenant un groupe méthoxycarbonyle en tant que groupe R¹, dans lequel on utilise du méthanol en tant qu'alcool.
